(19)

Europäisches Patentamt

European Patent Office

Office européen des brevets

(11) **EP 0 956 878 A2**

(12) **EUROPEAN PATENT APPLICATION**

(43) Date of publication:
**17.11.1999 Bulletin 1999/46**

(51) Int Cl.⁶: **A61M 25/00, A61M 25/06**

(21) Application number: **99303757.1**

(22) Date of filing: **14.05.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **14.05.1998 JP 13244098**
**10.03.1999 JP 6389799**
**10.03.1999 JP 6389999**

(71) Applicant: **TERUMO KABUSHIKI KAISHA**
**Tokyo (JP)**

(72) Inventors:
• **Tachikawa, Kouichi**
  **Nakakoma-gun, Yamanashi (JP)**
• **Okayama, Tomoki**
  **Nakakoma-gun, Yamanashi (JP)**

(74) Representative:
**Harrison, David Christopher et al**
**MEWBURN ELLIS**
**York House**
**23 Kingsway**
**London WC2B 6HP (GB)**

(54) **Intravascular catheter and catheter-needle assembly**

(57)    An intravascular catheter, an intravascular catheter assembly including such an intravascular catheter, and their production method are provided. The intravascular catheter is provided with a catheter main segment which has excellent resistance to breakage, kink and other damages simultaneously with adequate flexibility, workability of the tip portion, and surface smoothness. In addition, such catheter main segment may be optionally provided with radiopacity with no substantial increase in its wall thickness.

The intravascular catheter of the present invention is the catheter used in combination with an inner needle (stylet) in its piercing. The inner needle which had been inserted in the lumen of the catheter is removed after the piercing of the catheter to leave the catheter indwelled in the blood vessel. Therefore, the intravascular catheter has a flexible catheter main segment 2. The catheter main segment 2 comprises an inner layer 4 and an outer layer 6 which are adhered to each other with the intervening reinforcement layer 5. Preferably, the reinforcement layer comprises a braid of reinforcement yarns 51 of a thermoplastic resin, which is produced by winding the reinforcement yarn(s) in spiral fashion in two directions on the exterior surface of the inner layer. The resistance to breakage and kink and the flexibility are ensured at once by adequately selecting the mode of the winding. In addition, thin wall thickness and surface smoothness of the catheter main segment are ensured by partly embedding the reinforcement yarn 51 in the inner layer 4. Preferably, such partial embedding of the reinforcement yarn 51 is realized by a preliminary heating at a predetermined temperature of the reinforcement yarn 51 after its provision on the surface of the inner layer 4 and before the formation of the outer layer 6.

EP 0 956 878 A2

**Description**

BACKGROUND OF THE INVENTION

This invention relates to an intravascular catheter which is indwelled in an artery or vein during infusion, blood transfusion, and the like. This invention also relates to an intravascular catheter assembly.

An intravascular catheter assembly comprising a catheter (indwelling catheter) and a needle (stylet) is used for infusion of a solution into a patient. Such intravascular catheter assembly has a dual needle structure wherein the needle is inserted into the lumen of the catheter to form the assembly. In use, the assembly pierces the artery or the vein, and upon withdrawal of the interior needle, the catheter remains in place in the artery or in the vein. Therefore, the catheter main segment (body) is required to have an appropriate flexibility to realize the trackability required in following the bends and curves of the blood vessel.

The needle of the intravascular catheter assembly comprises a metal material such as stainless steel, and the needle has a sharp edge (tip). Therefore, insertion or withdrawal of the needle into or from the catheter is associated with some risk of damage such as cutting, breakage, shaving, cracking, snapping, peeling, or fracturing of the catheter main segment by the sharp edge, and such breakage of the catheter in the blood vessel is not least undesirable. The catheter main segment is also required to have kink resistance to avoid folding and kink at the curves and bends of the blood vessel, and workability of the distal end portion in the production. The catheter should also have a reduced wall thickness to reduce the damage of the blood vessel. Accordingly, not only the flexibility but also adequate strength and break resistance are required for the catheter.

In addition, the catheter which is to be indwelled in the blood vessel of the patient for a quite long period should have a smooth surface to thereby reduce the sliding resistance upon piercing and during the indwelling of the catheter.

As a countermeasure for the case if the catheter should get damaged and the broken pieces should remain in the body, an intravascular catheter wherein a radiopaque substance is provided on a part of the inner or the exterior surface of its main segment in spiral, annular, or linear form has been produced to facilitate the search of such broken pieces. Such intravascular catheter, however, was unfavorable since the radiopaque substance was exposed to detract from surface smoothness and safety. In view of such situation, there is a demand for an intravascular catheter which has sufficient surface smoothness and safety, and which has a thin wall despite its inclusion of the radiopaque substance.

SUMMARY OF THE INVENTION

An object of the present invention is to provide an intravascular catheter which has an appropriate flexibility as well as kink resistance, workability of the tip portion, and breakage resistance, and which has a thin wall despite its radiopacity. Another object of the invention is to provide an intravascular catheter assembly which includes such an intravascular catheter, and a method for producing such an intravascular catheter.

Such objects of the present invention are achieved by the (1) to (15) as described below.

(1) An intravascular catheter having a tubular, flexible catheter main segment wherein said catheter main segment comprises an inner layer, a reinforcement layer formed from reinforcement yarns disposed on the inner layer, and an outer layer, wherein

said catheter main segment has at least a part wherein said inner and outer layers are adhered to each other via said reinforcement layer, and
said reinforcement layer comprises a braid of reinforcement yarns wherein the yarns are wound in spirals of two directions, and wherein the relation:

$$n \times m > S$$

is met when n is the number of the reinforcement yarns wound in one direction,
m is the number of the reinforcement yarns wound in another direction, and
S is the total number of intersecting points in the braid within one full turn of the reinforcement yarn.

(2) An intravascular catheter having a tubular, flexible catheter main segment wherein said catheter main segment comprises an inner layer, a reinforcement layer formed from reinforcement yarns disposed on the inner layer, and an outer layer, wherein

said catheter main segment has at least a part wherein said inner and outer layers are adhered to each other via said reinforcement layer, and

said reinforcement yarns are partly embedded in the inner layer of said catheter main segment in the cross section perpendicular to axial direction of said catheter.

(3) An intravascular catheter having a tubular, flexible catheter main segment wherein said catheter main segment comprises an inner layer, a reinforcement layer formed from reinforcement yarns disposed on the inner layer, and an outer layer, wherein

said catheter main segment has at least a part wherein said inner and outer layers are adhered to each other via said reinforcement layer, and
the relation:

$$Tt > Ti + 5°C$$

is met when
Tt is melting temperature of said reinforcement yarns, and
Ti is the temperature at which the material constituting said inner layer reaches down to a melt viscosity of 100,000 poise.

(4) An intravascular catheter according to any one of the above (1) to (3) wherein said flexible material is a thermoplastic elastomer.
(5) An intravascular catheter according to the above (4) wherein said thermoplastic elastomer is selected from polyamide elastomers and polyurethane elastomers.
(6) An intravascular catheter according to any one of the above (1) to (5) wherein said reinforcement yarn comprises a member selected from polyamide thermoplastic resins, polyester thermoplastic resins, and mixtures of such resins with an inorganic material.
(7) An intravascular catheter according to any one of the above (1) to (6) wherein the exterior surface of the outer layer has a surface roughness Rz (measured in accordance with JIS) of up to 7 μm.
(8) An intravascular catheter according to any one of the above (1) to (7) wherein said catheter main segment has an inner diameter of about 0.4 to about 1.8 mm, an outer diameter of about 0.6 to about 2.3 mm, and a total wall thickness of about 0.08 to about 0.5 mm.
(9) An intravascular catheter according to the above (2) wherein 30 to 80% in total in cross sectional area of the reinforcement is embedded in the inner layer.
(10) An intravascular catheter according to the above (3) wherein said reinforcement yarns contain a radiopaque substance.
(11) An intravascular catheter according to the above (1) wherein the number of the reinforcement yarns is such that $n \geq 3$ and $m \geq 3$.
(12) An intravascular catheter according to the above (1) wherein the number of the reinforcement yarns is such that $n = m$.
(13) An intravascular catheter according to the above (1) wherein the number S of intersecting points in one turn of the reinforcement yarn is preferably such that $n \times m > 1.5 \times S$.
(14) A method for producing the intravascular catheter according to any one of the above (1) to (3) comprising the steps of forming the inner layer of tubular form, providing the reinforcement layer comprising the reinforcement yarns on the exterior surface of said tubular inner layer, and coating the exterior surface of the inner layer and the reinforcement layer with the material of the outer layer in molten state, wherein, before such coating, the tubular inner layer having the reinforcement layer provided thereon is preliminarily heated to a degree such that the reinforcement yarn retains its shape.
(15) An intravascular catheter assembly comprising the intravascular catheter according to any one of the above (1) to (13) and a needle inserted in said intravascular catheter.

BRIEF DESCRIPTION OF THE DRAWINGS

FIG. 1 is a plan view showing an embodiment of the intravascular catheter assembly of the present invention.
FIG. 2 is a partially cut away cross sectional view of the catheter main segment of the intravascular catheter shown in FIG. 1, showing its interior structure.
FIG. 3 is a partially cut away perspective view of the catheter main segment of the intravascular catheter shown in FIG. 1, showing the reinforcement layer structure thereof.

FIG. 4 is a partially cut away perspective view of the catheter main segment showing another embodiment of the reinforcement layer of the present invention.

FIG. 5 is a partially cut away perspective view of the catheter main segment showing further embodiment of the reinforcement layer of the present invention.

FIG. 6 is an expanded view of the reinforcement layer of grid pattern shown in FIG. 3.

FIG. 7 is a cross sectional view of an embodiment of the present invention showing how the reinforcement layer 5 is embedded in the intravascular catheter of the present invention.

FIG. 8 is a cross sectional view showing the interior structure according to an embodiment of the reinforcement yarn including a radiopaque substance of the present invention.

FIG. 9 is a cross sectional view showing the interior structure according to another embodiment of the reinforcement yarn including a radiopaque substance of the present invention.

DETAILED DESCRIPTION OF THE INVENTION

The present invention provides a catheter which has a tubular, flexible catheter main segment wherein a reinforcement layer is embedded in the catheter main segment.

The catheter main segment is preferably of the constitution which has a portion wherein an inner layer and an outer layer are adhered to each other and an intervening reinforcement layer is provided between the inner and the outer layers.

The intravascular catheter and the intravascular catheter assembly of the present invention are hereinafter described in detail by referring to the preferred embodiments shown in the drawings.

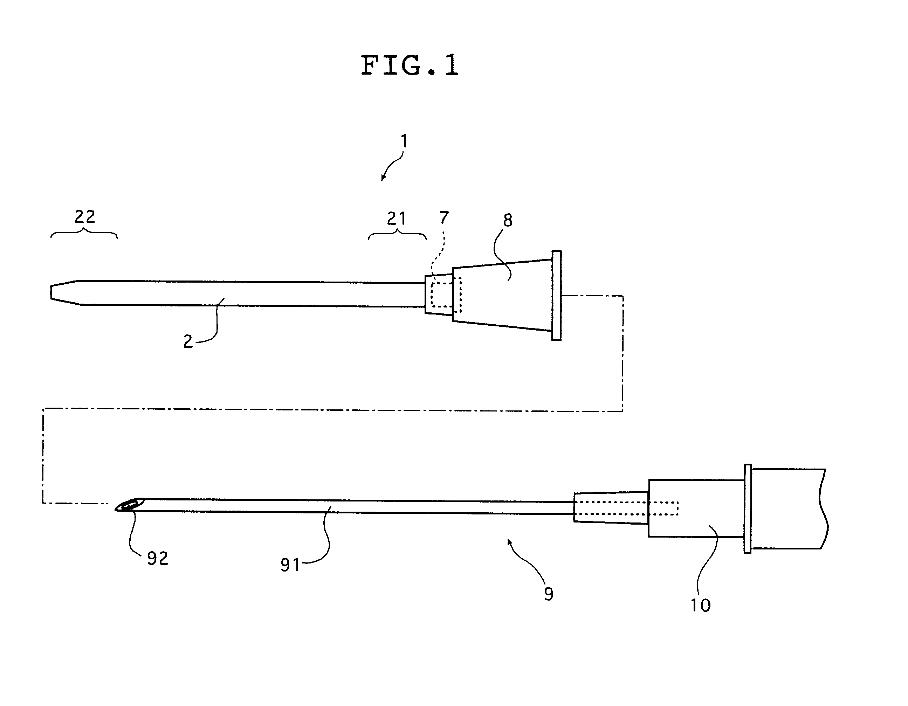

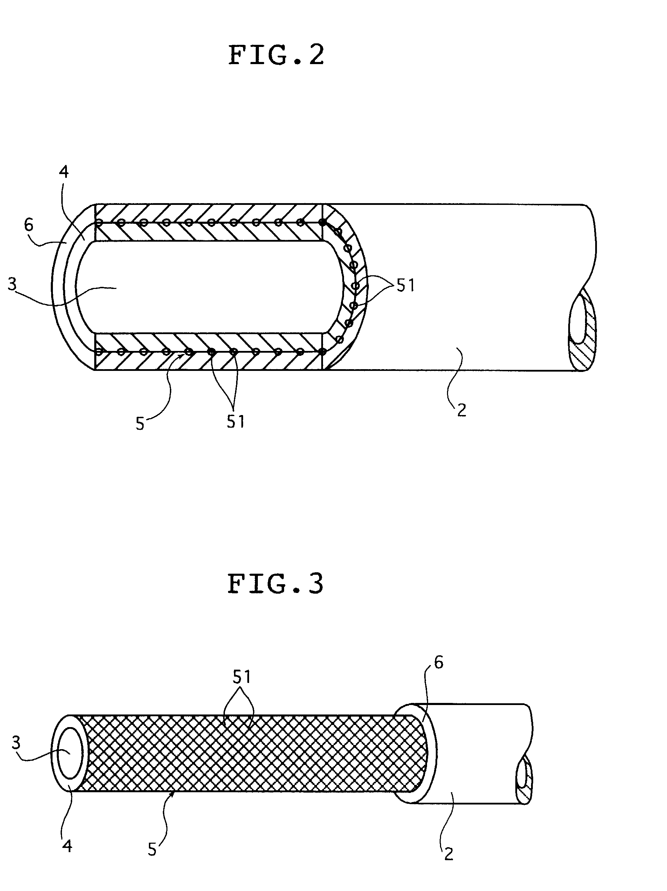

FIG. 1 is a plan view showing an embodiment of the intravascular catheter assembly of the present invention, and FIG. 2 is a partially cut away view of the catheter main segment of the intravascular catheter shown in FIG. 1, showing its interior structure in the intermediate portion. It should be noted that FIGS. 1 and 2 are schematic views wherein the catheter shown is magnified particularly in its radial direction.

As shown in FIG. 1, the intravascular catheter 1 of the present invention comprises the catheter main body 2 having a proximal end portion 21 and a distal end portion 22; a hub 8 secured to the proximal end portion 21 of the catheter main body 2; a crimp pin 7 which is crimped onto the proximal end portion 21 of the catheter main body 2 for fixture with the hub 8.

The catheter main segment 2 has a lumen 3 formed throughout its length from the proximal end portion 21 to the distal end. This lumen 3 serves the flow path of the fluids such as an infusion solution. When the intravascular catheter 1 is pierced into the blood vessel, a needle main segment 91 of a needle 9 as will be described below is preliminarily inserted in the lumen 3 of the catheter main segment 2 to form the intravascular catheter assembly.

Interior of the hub 8 is in communication with the lumen 3, and is connected to the proximal end of the lumen 3 to serve the role of port for injection of the fluid such as infusion solution or collection of the blood. The hub 8 also serves a handle for the operation of the intravascular catheter 1.

The crimp pin 7 is a tubular metal member which is dilated at its distal end. By fitting the crimp pin 7 in the hub 8, the proximal end portion 21 of the catheter main segment 2 is pressed against the distal end inner surface of the hub 8, and the catheter main segment 2 is fixedly secured to the hub 8.

The needle 9 comprises the needle main segment 91 having an acute edge (tip) 92 at its distal end and a needle hub 10 fixedly secured to the proximal end of the needle main segment 91. Preferably, the needle hub 10 fluid tightly fits in the hub 8. Such needle 9 and the catheter 1 constitute the intravascular catheter assembly of the present invention.

FIG. 2 shows inner structure of the catheter main segment 2.

As shown in FIG. 2, the catheter main segment 2 comprises an inner layer 4 and an outer layer 6 which are adhered to each other, and an intervening reinforcement layer is provided between the inner layer 4 and the outer layer 6. At least one, and preferably both of the inner layer 4 and the outer layer 6 comprises a flexible (soft) material.

Exemplary materials which may be used for the inner layer 4 and the outer layer 6 include polyolefins such as polypropylene, polyethylene, and ethylene-vinylacetate copolymer; polyamides; polyesters such as polyethylene terephthalate and polybuthylene terephthalate; polyurethane; polyvinyl chloride; polystyrene resins; fluororesins such as ethylene-tetrafluoroethylene copolymer; thermoplastic elastomers such as polyamide elastomers, polyester elastomers, polyurethane elastomers, polystyrene elastomers, and fluoroelastomers, which may be used alone or in combination or two or more.

Among these, the preferred are thermoplastic elastomers which are provided with high antithrombogenicity, extrudability, workability, kink resistance, economic properties, and the like.

The term "thermoplastic elastomer" is used herein as a concept including block copolymers of hard and soft segments; polymer alloys (including polymer blends and graft polymerized and random polymerized resins) of hard segment such as a polyamide and a flexible resin; polyamides softened by a plasticizer; and mixtures thereof.

Typical examples of such thermoplastic elastomers include polyamide elastomers. Exemplary polyamide elastomers are block copolymers prepared by using an aliphatic or aromatic polyamide, for example, nylon 6, nylon 64, nylon 66, nylon 610, nylon 612, nylon 46, nylon 9, nylon 11, nylon 12, N-alkoxymethyl modified nylon, hexamethylenediamine-isophthalic acid condensate, or metaxyloyldiamine-adipic acid condensate for the hard segment, and a polymer such as a polyester or a polyether for the soft segment.

Exemplary polyurethane elastomers include Miractoran (grades: E564PNAT, E568 PNAT, E574 PNAT, E998 PNAT) manufactured by Nippon Miractoran K.K. and exemplary polyester elastomers include Hytrel (Grade: 6377, 7277) manufactured by Toray-DuPont.

Among such thermoplastic elastomers, the preferred are polyamide elastomers and polyurethane elastomers in view of their antithrombogenicity, extrudability, workability, kink resistance, and economic properties.

The inner layer 4 and the outer layer 6 may comprise the same material or different materials. Use of the same material is preferred in view of extrusion convenience and adhesiveness.

Irrespective of whether the inner layer 4 and the outer layer 6 comprise the same material or different materials, the material used for the outer layer 6 may preferably have a Shore D hardness of about 30 to about 80, and more preferably, about 40 to about 70. When the outer layer comprises a material with such hardness range, the catheter main segment 2 will be provided with both adequate strength and adequate flexibility, and hence, with improved resistance to breakage and kink. For similar reason, the material used for the inner layer 4 may preferably have a Shore D hardness of about 30 to about 80, and more preferably, about 40 to about 70.

Thickness of the inner layer 4 and the outer layer 6 is not critical although the inner layer 4 may preferably have a thickness of about 0.02 to about 0.10 mm, and more preferably, about 0.02 to about 0.07, and the outer layer 6 may preferably have a thickness of about 0.02 to about 0.10 mm, and more preferably, about 0.03 to about 0.08.

The catheter main segment may have an inner diameter of about 0.4 to about 1.8 mm, an outer diameter of about 0.6 to about 2.3 mm, and a total wall thickness of about 0.08 to about 0.5 mm, preferably about 0.08 to about 0.25 mm.

In the embodiments shown in the drawings, the inner layer 4 and the outer layer 6 respectively have constant inner and outer diameters. The inner and the outer diameters of the layers, however, may vary along the longitudinal axis of the catheter main body 2. For example, the inner diameter of the inner layer 4 may gradually increase toward the distal end. Alternatively, the outer diameter of the inner layer 4 or the outer layer 6 may gradually decrease toward the distal end in the distal end portion 22 of the catheter main segment 2.

As described above, the reinforcement layer 5 is provided at the boundary between the inner layer 4 and the outer layer 6. The configuration of the reinforcement layer 5 is not critical, and the reinforcement layer 5 may comprise either a continuous or a non-continuous structure of the reinforcement layer sheet or the reinforcement yarn.

Among these, the preferred is continuous reinforcement layer structures of reinforcement yarns. Cross sectional shape of the reinforcement yarn is not critical, and exemplary shapes include circle, oval, oblong, triangle, rectangle, square, and star shape. Among these, the preferred are the reinforcement yarns having a circular cross section.

Next, it is mainly described that the configuration of the reinforcement layer is the reinforcement yarn.

The material for the reinforcement yarn 51 of the reinforcement layer 5 may be any material as long as the material has rigidity sufficient for providing the catheter main segment 2 with satisfactory resistance to breakage (cutting) and kink. Preferably, the material is a thermoplastic resin, and the thermoplastic resin may be the one which has a thermoplasticity sufficient to become molten or softened when it is heated in the course of shaping the tip portion of the distal end portion 22 of the catheter main segment 2 into the desired shape (for example, tapered shape as shown in FIG. 1).

Exemplary such reinforcement materials include polyesters such as polyethylene terephthalate (PET) and polybutylene terephthalate (PBT), polyolefins such as polyethylene and polypropylene, hard polyvinyl chlorides, polyamides, polyimides, polyamide imides, polystyrenes, thermoplastic polyurethanes, polycarbonates, ABS resins, AS resins, acrylic resins, polymethyl methacrylate (PMMA), polyacetal (PA), polyarylates, polyoxymethylene (POM), high tension polyvinyl alcohols, fluororesins, polyvinylidene fluorides (PVdF), polytetrafluoroethylene, saponified ethylene-vinyl acetate (EVOH), polysulfone, polyether sulfone, polyether ketone, polyether ether ketone, polyphenylene oxide, polyphenylene sulfide, liquid crystal polymers, polymer alloys containing at least one of the foregoing, and combinations of two or more of the foregoing. Among these, the most preferred are polyamides, polyamide resins containing a polyamide as their main component, and polyesters.

The term "polyamide" used herein is a concept including aliphatic and aromatic polyamides, for example, nylon 6, nylon 64, nylon 66, nylon 610, nylon 612, nylon 46, nylon 9, nylon 11, nylon 12, N-alkoxymethyl modified nylon, hexamethylenediamine-isophthalic acid condensate, and metaxyloyldiamine-adipic acid condensate; and polymer alloys (polymer blends, graft or random polymerized resins, etc.) of such polyamide with other resins; and mixtures thereof.

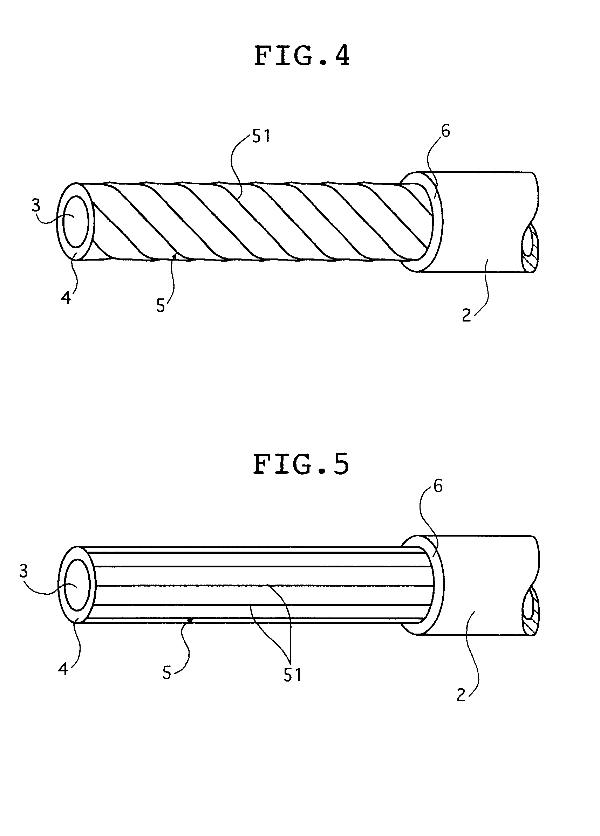

FIGS. 3, 4 and 5 are perspective views showing various embodiments of the reinforcement layer 5.

In the embodiments of FIGS. 3, 4 and 5, the reinforcement layer 5 comprises the reinforcement yarns 51.

In the embodiment shown in FIG. 3, the reinforcement layer 5 comprises a braid or grid of the reinforcement yarns 51 formed on the exterior surface of the inner layer 4. Such braid of the reinforcement yarns 51 is formed by winding at least one reinforcement yarn 51 in spirals of two different directions.

In the embodiment shown in FIG. 4, the reinforcement layer 5 comprises at least one reinforcement yarn 51 wound in spirals on the exterior surface of the inner layer 4. The reinforcement yarns 51 are wound in one direction at a predetermined pitch.

In the embodiment shown in FIG. 5, the reinforcement layer 5 comprises the reinforcement yarns 51 extending along longitudinal axis of the catheter main segment 2 on the exterior surface of the inner layer 4.

It should be noted that the pattern of the reinforcement yarns 51 may be a combination of those shown in FIGS. 3, 4 and 5, or the one other than those shown in FIGS. 3, 4 and 5.

By constituting the reinforcement layer 5 from the reinforcement yarns as described above, the resulting catheter main segment 2 will have a reduced wall thickness, an improved resistance to breakage and kink, as well as a sufficient flexibility.

Among the embodiments described above, the preferred is the embodiment of FIG. 3 wherein the reinforcement yarns 51 are braided by winding the yarns in spirals of two different directions.

As a countermeasure for breakage of the catheter main segment and remaining of the broken piece in the body, the reinforcement yarns 51 are preferably braided such that each yarn wound in one direction passes over and under each successive yarn wound in another direction.

In such a case, the resulting reinforcement layer 5 will have a sufficient flexibility if the relation:

$$n \times m > S$$

is met when n is the number of reinforcement yarns wound in one direction, m is the number of reinforcement yarns wound in another direction, and S is the total number of intersecting points in the braid within one full turn of the reinforcement yarn.

The number of reinforcement yarns 51 is preferably such that $n > 3$ and $m > 3$, and more preferably that $n = m$.

The number of intersecting points in one turn of the reinforcement yarn is preferably such that $n \times m > 1.5 \times S$.

In the embodiment of the catheter main segment 2 shown in FIG. 3, the braid comprises 8 reinforcement yarns 51 wound in one direction and 8 reinforcement yarns 51 wound in another direction, and the number of intersecting points S in the braid within one turn of any reinforcement yarns 51 is 32.

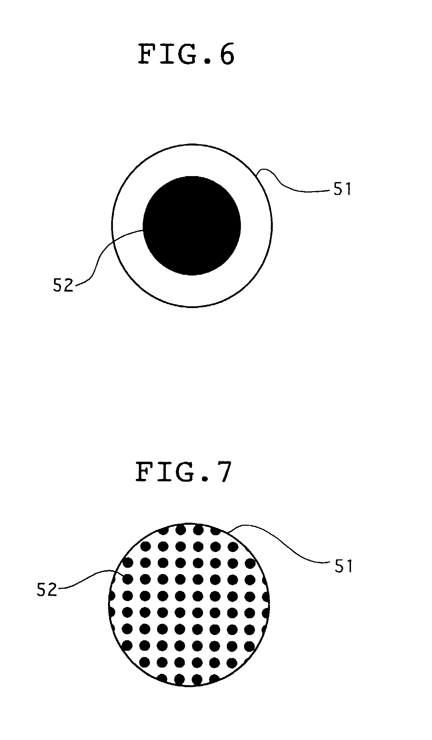

FIG. 6 is an enlarged view showing another embodiment of the braid wherein the number of intersecting points S is 32. In this embodiment, two adjacent reinforcement yarns 51a wound in one direction float over two adjacent reinforcement yarns 51b wound in another direction and sinks under the next two adjacent reinforcement yarns 51b wound in another direction, and this over-under-over-under passing is repeated. The two adjacent reinforcement yarns 51a adjacent the above-described two reinforcement yarn 51a also repeats the over-under-over-under passing except that the pattern is shifted by two yarns. In this embodiment, the term "point of intersection" means the site where the two adjacent reinforcement yarns 51b wound in one direction intersect with (i.e. float over or sink under) the two adjacent reinforcement yarns 51b wound in another direction.

The present invention is not limited to the embodiments as described above, and the reinforcement layer 5 may comprise, for example, a braid wherein the number of intersecting points S is 16.

Among the embodiments as described above, the reinforcement layer 5 is preferably the one wherein the reinforcement yarns 51 are braided as in the case of FIGS. 3 and 6 since the resulting intravascular catheter has sufficient flexibility simultaneously with excellent resistance to kink and breakage. Such intravascular catheter will also have a sufficiently thin wall thickness even if a radiopaque substance is included. As a consequence, the intravascular catheter will retain its trackability at the curves and bends of the blood vessel, and the catheter will also have a sufficient kink resistance to resist folding at such curves and workability of tip portion in the course of its production.

In the present invention, the reinforcement yarn(s) 51 may be arranged either at a constant density or at varying density along the catheter main segment 2. For example, the reinforcement yarns 51 may be provided at a higher density in the portion where higher rigidity is required (for example in the distal end portion 21) and at a loser density in the portion where higher flexibility is required (for example in the proximal end portion 22). Alternatively, the reinforcement yarns 51 may be provided at a higher density in the distal end portion 22 in view of the required resistance to breakage or cutting.

In the embodiments of FIGS. 3 and 4, such density of the reinforcement yarns 51 can be adjusted by at least one of pitch of the reinforcement yarns 51 wound in spirals (i.e. interval between two adjacent reinforcement yarns 51), number of the reinforcement yarns 51, and angle θ between the reinforcement yarns 51 and the longitudinal axis of the catheter main segment 2 as described below. In the embodiment of FIG. 5, such density may be adjusted by adjusting the number of the reinforcement yarns 51.

The angle θ between the reinforcement yarns 51 and the longitudinal axis of the catheter main segment 2 (hereinafter simply referred to as the tilt angle) is preferably in the range of 0 to 70°, more preferably 15 to 65°, and most preferably 15 to 60°. The situation where the tilt angle θ equals 0° corresponds to the embodiment of FIG. 5 wherein the reinforcement yarns 51 are longitudinally applied in parallel with the longitudinal axis of the catheter main segment 2. When the tilt angle θ is within the above specified range, the resulting catheter main segment 2 will have improved kink resistance and trackability.

The tilt angle θ as described above may be constant along the full length of the reinforcement layer 5. Alternatively, reinforcement layer 5 may comprise two or more segments each having different tilt angle. The pitch is preferably in the range of 1 to 7 mm.

In addition, the reinforcement layer 5 may not necessary extend along the full length of the catheter main segment 2, and the catheter main segment 2 may have some regions where the reinforcement layer 5 is absent. For example, the reinforcement layer 5 may extend along the full length of the catheter main segment 2 except for the distal end portion 22 or the proximal end portion 21. For improving the breakage resistance of the distal end portion 22, the reinforcement layer 5 (reinforcement yarns 51) is preferably provided at the distal end portion 22.

The reinforcement yarn 51 may comprise either a single filament or a filament bundle (e.g. a strand of filaments). Use of the reinforcement yarn 51 in the form of a single filament is more preferable in view of the easiness of shaping the distal end portion 22.

The thickness (diameter) of the reinforcement yarn 51 is determined in relation to the material employed. In other words, the reinforcement yarn 51 may have an adequate diameter so that the catheter main segment 2 will have a sufficient stiffness as well as sufficient flexibility, and the distal end portion 22 can be worked into the desired shape. For example, when the reinforcement yarn 51 comprises a single filament of polyamide, the diameter is preferably in the range of about 20 to about 50 μm. The filament may be used as a single filament or as a bundle of filaments.

In the embodiments of FIGS. 3 to 5, the reinforcement layer 5 comprises the same type of reinforcement yarns 51. The present invention is not limited to such embodiments, and two or more types of reinforcement yarns 51 may be used in combination. For example, when the reinforcement layer 5 contains a radiopaque substance, the reinforcement layer 5 may comprise single type of reinforcement yarns 51 containing the radiopaque substance. The present invention, however, is not limited to such embodiments and the reinforcement layer 5 may comprise two or more types of reinforcement yarns 51 at least one of which containing the radiopaque substance.

In the reinforcement layer 5 as described above, and in particular, in the reinforcement layer 5 comprising the reinforcement yarns 51, the reinforcement layer 5 may constitute about 0.1 to about 30%, preferably about 0.5 to about 25%, and more preferably about 3 to about 20% of said catheter main segment in cross sectional area. When the area ratio of the reinforcement layer 5 is too low, the effect of providing the reinforcement layer may become insufficient depending on the combination of the materials used for the reinforcement layer 5, the inner layer 4 and the outer layer 6. When the area ratio the reinforcement layer 5 is too high, the flexural strength of the catheter main body 2 may become too high to detract from flexibility depending on the combination of the materials used for the reinforcement layer 5, the inner layer 4 and the outer layer 6.

As described above, the distal end portion 22 of the catheter main segment 2 may be tapered by heat treatment, and the proximal end portion 21 of the catheter main segment 2 may be also heat treated into trumpet shape for fitting of the crimp pin 7. In such occasion, it is preferable that the reinforcement yarn 51 constituting the reinforcement layer 5 also soften with the inner and outer layers 4 and 6.

More illustratively, the difference in melting temperature or the difference between the softening temperature between the reinforcement yarn 51 (constituting the reinforcement layer 5) and the inner and outer layers 4 and 6 (an average of the inner and outer layers 4 and 6 if they comprise different materials) is up to 60°C, and preferably up to 30°C.

When materials of the inner and outer layers 4 and 6 and the reinforcement layer 5 (the reinforcement yarn 51) satisfy the thermal conditions as described above, the resulting catheter main segment will be the one wherein the reinforcement yarn 51 is not melted and wherein the reinforcement yarn 51 is partly embedded in the inner layer when the catheter is fabricated by the method as will be described later.

FIG. 7 is a cross sectional view of an embodiment of the catheter main segment 2 in the plane perpendicular to the longitudinal axis of the catheter main segment 2, showing in magnified view how the reinforcement layer 5 is partly embedded in the inner layer.

In the cross section of the catheter main segment 2 in the plane perpendicular to the longitudinal axis of the catheter main segment 2, the reinforcement layer 5 is preferably embedded in the inner layer 4 to a degree such that the total cross sectional area of the reinforcement layer 5 embedded in the inner layer 4 on the average is in the range of 30 to 80%, and more desirably approximately 50% of the total cross sectional area of the reinforcement layer 5.

When the degree of embedding of the reinforcement layer 5 in the inner layer 4 is up to 20%, the degree of embedding of the reinforcement layer 5 in the outer layer 6 would be quite high and the grid pattern of the reinforcement layer 5 may become apparent as surface irregularities on the exterior surface of the catheter main segment 2 when the thickness of the inner layer 4, the outer layer 6, the catheter main segment 2, and the reinforcement yarn 51 are within the above described ranges. Production of a catheter main segment 2 free from such surface irregularities in grid pattern of the reinforcement layer 5 is quite difficult since the catheter main segment 2 is thin walled and long. The pattern of the reinforcement layer 5 as surface irregularities of the catheter main segment 2 or the reinforcement layer 5 itself appearing on the exterior surface of the catheter main segment 2 may induce pain in the piercing or indwelling of the catheter.

When the degree of embedding of the reinforcement layer 5 in the inner layer 4 is 80% or more, the degree of embedding of the reinforcement layer 5 in the inner layer 4 would be quite high and the grid pattern of the reinforcement layer 5 may become apparent as surface irregularities in the interior surface of the catheter main segment 2 on the side of the lumen 3 when the thickness of the inner layer 4, the outer layer 6, the catheter main segment 2, and the reinforcement yarn 51 are within the above described ranges. Production of a catheter main segment 2 free from such surface irregularities in grid pattern of the reinforcement layer 5 is quite difficult since the catheter main segment 2 is thin walled and long. The pattern of the reinforcement layer 5 as surface irregularities of the catheter main segment 2 or the reinforcement layer 5 itself protruding into the lumen 3 may result in such situation that, upon insertion of the needle main segment 91 into the lumen 3, the protruding parts of the inner layer 4 or the reinforcement layer 5 protruding into the lumen 3 is pushed by the inserted needle main segment 91 to result in the surface irregularities in the grid pattern on the exterior surface of the outer layer 6, or shaving of the protruding parts of the inner layer 4 or the reinforcement layer 5 protruding into the lumen 3 by the inserted needle main segment 91. Furthermore, in the embedding of the reinforcement layer 5 in the inner layer 4, the inner layer 4 is likely rise and protrude into the outer layer 6 at the gaps defined between the inner surface 4 and the braided reinforcement layer 5 and the grid pattern of the reinforcement layer 5 is likely to become apparent as surface irregularities on the exterior surface of the catheter main segment 2.

On the other hand, the outer diameter of the catheter main segment becomes larger as the outer layer 6 is thicker to get a smooth surface.

In the present invention, the relation:

$$Tt > Ti$$

should be met when

Tt is melting temperature of the material of said reinforcement layer (yarn), and
Ti is the temperature at which melt viscosity of the material constituting the inner layer reaches down to 100,000 poise. It is more preferable that the relation: $Tt > Ti + 5°C$ is met, and most preferable that the relation: $Tt > Ti + 10°C$ is met.

It should be noted that poise is a viscosity unit which can be converted by the following equation:

$$1 \text{ poise} = 10^{-1} N·s /m^2 \text{ (N stands for Newton)}$$

The reinforcement layer 5 of the present invention may contain a radiopaque substance as described above.

Any radiopaque substance may be used for the radiopaque substance 52 included in the reinforcement layer 5 as long as the radiopacity is sufficient. Exemplary radiopaque substances include, barium sulfate, bismuth oxide, bismuth bicarbonate, powder tungsten, powder tantalum, and mixtures thereof.

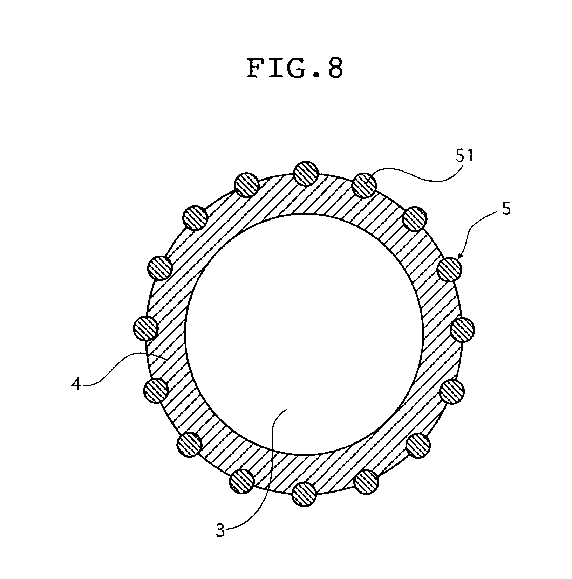

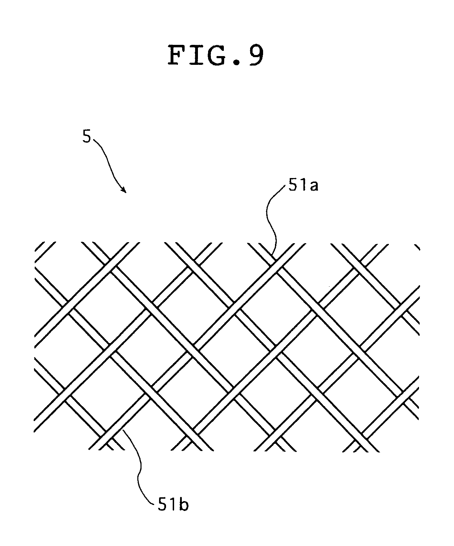

When the reinforcement layer 5 contains the radiopaque substance 52, the radiopaque substance 52 may be incorporated, for example, as the core of the reinforcement yarn 51, or in the form of finely divided powder dispersed in the reinforcement yarn 51. These embodiments are shown in FIGS. 8 and 9 in cross sections, respectively. In the latter case, the radiopaque substance 52 should be uniformly dispersed in the predetermined part of the reinforcement yarn 51, and the radiopaque substance 52 should have a sufficient dispersibility in the material of the reinforcement yarn 51, and therefore, use of the radiopaque substance as a finely divided powder having a particle size of 0.1 to 5 µm is preferable. Such reinforcement yarn 51 can be produced by conventional spinning process. In the former case wherein the radiopaque substance 52 is incorporated as the core of the reinforcement yarn 51, such reinforcement yarn 51 may be produced by a conventional process used for spinning core-sheath type complex filaments. In the latter case wherein the radiopaque substance 52 is incorporated as finely divided powder in the reinforcement yarn 51, such reinforcement yarn 51 may be produced by a conventional process using the blend of the radiopaque powder and the reinforcement layer material.

The reinforcement layer 5 provided in the catheter main segment 2 imparts improved breakage resistance to the catheter main segment 2 against the acute edge of the needle main segment 91. However, even if the breakage should occur, the broken piece includes the radiopaque substance 52 and the search of the broken piece by angiography is greatly assisted by the presence of such radiopaque substance 52. In addition, the radiopaque substance 52 is not exposed, and the catheter main segment 2 has excellent surface smoothness and safety.

In the embodiments of the intravascular catheter of the present invention wherein braided reinforcement yarns of a thermoplastic resin each containing the radiopaque material therein is used for the reinforcement layer 5, increase in the thickness of the catheter main segment 2 is avoided compared to the cases of intravascular catheters wherein a layer containing a radiopaque substance is disposed as an intermediate layer in the laminate. Despite such reduced thickness, sufficient radiopacity is still obtained since the reinforcement yarn 51 contains the radiopaque substance. If the reinforcement yarn 51 contains the radiopaque substance 52 in its core, good adhesion between the surface of the reinforcement yarn 51 and the inner and outer layers 4 and 6 are readily ensured.

In particular, in the preferred embodiment of the present invention wherein the reinforcement yarns 51 are partly embedded in the inner layer 4, the resulting catheter main segment 2 is provided with sufficient radiopacity as well as the reduced wall thickness and excellent surface smoothness.

In contrast, if the radiopaque substance-containing layer were disposed as the intermediate layer between the inner layer and the outer layer, thickness of the intermediate layer adds to the thickness of inner and outer layers and increase in the thickness of the catheter main segment 2 can not be avoided, and production process is also complicated.

The catheter main segment 2 wherein the reinforcement yarn 51 is partly embedded in the inner layer 4 with its yarn shape retained also has excellent exterior surface smoothness. The surface of the inner layer side is generally smooth since the catheter is generally produced by using a mandrel and the mandrel is withdrawn to leave the lumen.

In this invention, the surface roughness Rz (average of 10 points) is desirably up to 7 $\mu$m. In particular, up to 7 $\mu$m of the surface roughness Rz (average of 10 points) is possible when the catheter main segment has an inner diameter of about 0.4 to about 0.9 mm, an outer diameter of about 0.6 to about 1.2 mm, and a total wall thickness of about 0.087 to about 0.17 mm. Furthermore the surface roughness Rz (average of 10 points) is desirably up to 5 $\mu$m, more desirably up to 4 $\mu$m when the catheter main segment has an inner diameter of about 0.9 to about 1.8 mm, an outer diameter of about 1.2 to about 2.3 mm. The surface roughness Rz (average of 10 points) is measured in accordance with JIS. More illustratively, the surface roughness Rz is calculated for a standard length of the surface profile, as a difference in micrometer ($\mu$m) between average height of the highest to the fifth highest peaks and average height of the bottoms of the deepest to the fifth deepest valleys where the height is measured in transverse direction from a line which is parallel to the mean line and which does not intersect the surface profile

As described above, since the intravascular catheter 1 of the present invention which has the reinforcement layer 5, and in particular, the reinforcement layer 5 comprising the reinforcement yarns 51, the catheter main segment 2 has an adequate flexibility simultaneously with improved resistance to breakage (resistance to damaging, shaving, cracking, snapping, peeling, cutting, and fracturing) and kink resistance. The intravascular catheter 1 of the present invention also has an improved workability of the distal end portion 22 since the reinforcement layer 5 is fabricated from an adequately selected material.

It should be noted that the intravascular catheter of the present invention is not limited to the embodiments as shown in the drawings, and those wherein the boundary between the inner layer 4 and the outer 6 is absent or unclear, and those wherein the inner layer 4 and the outer 6 are integrated are also within the scope of the invention.

Next, the method for producing the intravascular catheter 1 as described above is described by referring to preferable embodiments.

The inner layer 4 of the catheter main segment 2 is first produced by standard method in the art, for example, by extrusion.

The reinforcement yarns 51 are then wound around the exterior surface of the inner layer in spirals, for example, by using an apparatus called spiral wrap machine (not shown). The reinforcement yarn 51 is supplied from the yarn supplying member of the spiral wrap machine, and the yarn is wound around the exterior of the inner layer 4 from one end by rotating the inner layer 4 around the longitudinal axis of the inner layer 4 in one direction and simultaneously moving the inner layer 4 along the longitudinal axis of the inner layer 4.

Upon reaching of the yarn 51 to the other end of the inner layer 4, the winding of the yarn 51 is continued by moving the inner layer 4 in longitudinally reverse direction without changing the direction of the rotation; or alternatively, winding is restarted from the starting end, and this time, rotating the inner layer 4 in the other direction. A braid of the reinforcement yarns 51 as shown in FIG. 3 is thereby produced.

In the embodiments as described above, a process is employed wherein the inner layer is rotated around the longitudinal axis, and simultaneously, moved along the longitudinal axis. The process is not limited such an embodiment and any process may be employed as long as the inner layer rotates in relation to the yarn supplying member, and the inner layer moves in the longitudinal direction in relation to the yarn supplying member. Exemplary such processes include (1) a process wherein the inner layer rotates around its longitudinal axis and the yarn supplying member moves along the longitudinal axis of the inner layer; (2) a process wherein the yarn supplying member rotates around the exterior of the inner layer and the inner layer moves along its longitudinal axis; and (3) a process wherein the inner layer is fixed to a predetermined position, and the yarn supplying member rotates around and moves along the longitudinal axis of the inner layer. Such processes may also be used in adequate combinations.

When the structure shown in FIG. 6 is formed by winding the reinforcement yarns 51 around the inner layer 4, the yarns are wound such that the yarn 51a wound in one direction always intersects with the yarn 51b wound in another direction to form a braid. This process may be accomplished with an apparatus called a braider (not shown), wherein the yarns 51 are continuously wound around the exterior surface of the inner layer 4 by rotating the yarn supplying members around the inner layer 4 with the yarns 51 supplied and moving the inner layer 4 along its longitudinal axis.

When the reinforcement layer 5 is provided on the exterior surface of the inner layer 4, the inner layer 4 having formed thereon the reinforcement layer 5 is covered by the outer layer 6 such that the inner surface of the outer layer 6 is firmly adhered to the exterior surface of the inner layer 4 and the reinforcement yarns 51.

Exemplary method of the outer layer formation include (a) adhesion of the outer layer 6 by means of an adhesive or a solvent; (b) fusion bonding of the outer layer 6 with the inner layer 4 (for example, by applying heat or radio frequency); (c) a process wherein the outer layer 6 is preliminarily formed and the preliminarily formed outer layer 6 is expanded by means of heat or a solvent, the inner layer 4 is inserted into the expanded outer layer, and the outer layer 6 is allowed to shrink; (d) a process wherein the material of the outer layer 6 in the form of a molten resin or a solution in an appropriate solvent is applied on the exterior surface of the inner layer 4 by dipping, coating or the like, and the thus applied material is solidified by cooling or drying (solvent removal) to thereby form the outer layer; and the like.

Among these, the most convenient and the one commonly adopted in the art is the process wherein the material of the outer layer 6 in the form of a molten resin is coated on the exterior surface of the inner layer 4, and the coating is solidified to form the outer layer 6.

In the production of the intravascular catheter 1, and in particular, in the production of a structure wherein the reinforcement yarns 51 are partly embedded in the inner layer 4 as shown in FIG. 7, the reinforcement layer 5 provided on the exterior surface of the inner layer 4 is embedded in the inner layer 4 to the degree of 30 to 80% in cross sectional area by allowing the reinforcement layer 5 to shrink or by applying pressure to the reinforcement layer 5.

Among these, the preferred is the method wherein the reinforcement layer 5 is allowed to shrink without applying any contact to the reinforcement layer 5 since loss of the mechanical strength and dislodgment of the reinforcement layer 5 can be avoided. In addition, the inner layer 4 is preferably softened at the time of the embedding of the reinforcement layer 5. For example, in the case of the reinforcement layer 5 comprising a polyamide, embedding of the reinforcement layer 5 in the inner layer 4 can be conveniently accomplished by applying heat to the inner layer 4 having provided thereon the reinforcement layer 5 to thereby soften the inner layer simultaneously with the shrinkage of the reinforcement layer 5. Displacement of the reinforcement layer 5 is prevented when the reinforcement layer 5 is embedded in the inner layer 4.

An intravascular catheter having improved surface smoothness is produced by the method comprising the steps of forming the inner layer of tubular form, providing the reinforcement layer comprising the reinforcement yarns on the exterior surface of the tubular inner layer, and coating the exterior surface of the inner layer and the reinforcement layer with the material of the outer layer in molten state, wherein, before such coating, the tubular inner layer having the reinforcement layer provided thereon is preliminarily heated to a degree at which the reinforcement yarn retains its shape.

In such preliminary heating, the temperature of preliminary heating $T_p$ and the melting temperature of the material of the reinforcement layer (yarn ) $T_t$ is preferably in the relation:

$$T_t > T_p$$

It is more preferable that the relation: $T_t > T_p + 5°C$ is met, and most preferable that the relation: $T_t > T_p + 10°C$ is met. When such relation is satisfied, softening of the inner layer 4 to a sufficient degree is realized without melting or reduction in strength of the reinforcement yarns 51 by the preliminary heating, and as a consequence, the reinforcement yarns 51 is adequately embedded in the inner layer and sufficient adhesiveness between the inner and outer layers are attained.

In addition, it is preferable when such preliminary heating is conducted that the relations:

$$To - 30°C < Tp < Ti + 10°C,$$

and

$$Tb < Tp < Tt$$

are met when

Tt is melting temperature of said thermoplastic reinforcement layer (yarn) material,
Tp is temperature of the preliminary heating,
Ti is the temperature at which melt viscosity of the material constituting the inner layer reaches down to 100,000 poise,
To is the temperature at which melt viscosity of the material constituting the outer layer reaches down to 100,000 poise, and
Tb is Vicat softening temperature (as determined by JIS K7206 under the load of 1 kg) of the inner layer.

The intravascular catheter 1 produced by the method wherein the relations as described above are satisfied are provided with the catheter main segment 2 which is radiopaque and which has a sufficiently thin wall thickness as well as an improved resistance to cutting and kink.

The Vicat softening point is measured by "liquid heating method" specified in JIS K7206 as the temperature of heat-conductive medium when a penetrator needle penetrates the specimen to a depth of 1 mm under the specified load of 1 kg.

The resulting laminate of the inner layer 4, the reinforcement layer 5, and the outer layer 6 is cut in predetermined length, and necessary workings of the distal end portion 22 and the proximal end portion 21 are conducted if such workings are desired. The catheter main segment 2 is thereby completed.

The crimp pin 7 is fitted on the resulting catheter main segment 2 at its proximal end portion 21, and the hub 8 is then secured to complete the intravascular catheter 1.

The intravascular catheter 1 and its production method is of course not limited to the embodiments as described above, and in particular, various parts constituting the intravascular catheter 1 can be replaced with other parts as long as such parts fulfil equivalent functions.

In addition to the inner layer 4, the reinforcement layer 5, and the outer layer 6, the catheter main segment 2 of the intravascular catheter 1 of the present invention may include members such as a covering layer, an intermediate layer, and the like necessary for the particular purpose. Exemplary such additional members include a protective layer, an antithrombogenic layer, a lubricating layer, an antiabrasive layer and the like on the exterior surface of the outer layer 6 or on the interior surface of the inner layer 4 on the side of the lumen 3, and an adhesive layer formed as an intermediate layer at any appropriate location between the inner layer 4 and the outer layer 6.

The intravascular catheter assembly wherein the catheter main segment of the present invention is used for the intravascular catheter can fulfil various functions required for an intravascular catheter assembly with no risk of the catheter main segment being damaged by the acute edge of the needle upon insertion of the needle main segment into the lumen of the catheter main segment.

Next, the present invention is described in further detail by referring to Examples of the present invention and Comparative Examples which by no means limit the scope of the present invention.

EXAMPLES

Example 1

The catheter main segment 2 shown in FIGS. 2 and 3 were fabricated basically by the procedure as described above. The reinforcement layer 5 was prepared by winding the reinforcement yarn 51 with a power-driven braider provided with yarn supplying members which supply the reinforcement yarns 51 to the exterior of the inner layer 4.

In forming the reinforcement layer 5, the yarn supplying members were rotated around the inner layer 4 in clockwise and counter clockwise directions, and simultaneous with such movements of the yarn supplying members, the inner layer 4 was moved in axial direction to thereby allow the reinforcement yarns 51 to be continuously wound around the exterior surface of the inner layer 4 at a constant tilt angle θ in relation to the longitudinal axis of the catheter main segment 2 and at a constant pitch. As a consequence, the reinforcement yarn 51a wound in one direction intersected with the reinforcement yarn 51b wound in another direction to form an intersecting point. By adequately setting the timing of intersecting of the yarn supplying members, the number of intersecting points at which the reinforcement yarn 51a wound in one direction intersected with the reinforcement yarn 51b wound in another direction was determined. In addition, by adequately setting the number of reinforcement yarns 51 and the speed of the longitudinal movement of the inner layer 4, number of the reinforcement yarns 51 in one pitch, namely, density of the reinforcement yarns 51 was determined.

Example 1-1

The catheter main segment 2 was produced in accordance with the specifications and the production conditions as described below.

Total length of the catheter main segment 2: 32 mm Outer diameter of the outer layer 6: 0.86 mm
Outer diameter of the inner layer 4: 0.75 mm
Inner diameter of the inner layer 4: 0.64 mm

Material of the outer layer 6: thermoplastic polyurethane
elastomer (Shore D hardness: 67, Melting point: 170°C)

Material of the inner layer 4: the one used for the outer layer (Melting point: 170°C)

Material of the reinforcement yarn 51: polyamide (nylon 6, melting point: 210°C), single filament
Diameter of the reinforcement yarn 51: 41 μm
Number of intersecting points of the braid in one full turn of the reinforcement yarn 51: 32 (the structure of FIG. 6)
Tilt angle θ of the reinforcement yarn 51 in relation to the longitudinal axis of the catheter main segment: 30°
Number of the reinforcement yarns 51: clockwise, 8; counter clockwise, 8; total, 16.

Before the coating of the material of the outer layer 6 in molten state, the surface of the reinforcement layer 5 was preliminarily heated to 160°C to adequately heat the reinforcement yarn 51. The material of the outer layer 6 was then coated, and the coating was cooled for solidification to thereby form the outer layer 6.
The resulting catheter main segment 2 was evaluated as described below.

Kink resistance

The catheter main segment 2 was bent at a radius of curvature of 5 mm to 90°C for 50 times, and then evaluated for the occurrence of the folding (kink). The measurement was conducted for 20 catheter main segments.

Breakage resistance

The catheter main body 2 was pulled at a rate of 300 mm/min in the direction of the longitudinal axis until breakage, and load at breakage was measured (tensile strength). The measurement was conducted for 20 catheter main segments at room temperature (20°C ), and average was calculated.

Flexibility

Opposite ends of the catheter main segment 2 were held by two hands and the catheter main segment 2 was bent by bringing the two hands together. The flexibility of the catheter main segment 2 of Examples and Comparative Example in the course of such bending was evaluated by the resistance felt in the bending according to the following relative criteria:

◎:   excellent
○:   good
×:   poor

The results were as shown below.

| Sample | Example 1-1 |
|---|---|
| Kink occurrence | 0/20 |
| Breakage load | 2.7 kg |
| Flexibility | ○ |

Example 1-2

The catheter main segment 2 was produced by repeating the procedure of Example 1-1 except that the number of reinforcement yarns 51 was reduced to 12 (6 clockwise yarns and 6 counter clockwise yarns). The evaluation was conducted as in Example 1-1. The results are shown below.

| Sample | Example 1-2 |
| --- | --- |
| Kink occurrence | 0/20 |
| Breakage load | 2.3 kg |
| Flexibility | ○ |

Example 1-3

The catheter main segment 2 was produced by repeating the procedure of Example 1-1 except that the number of intersecting points in the braid within one full turn of the reinforcement yarn 51 was reduced to 16. The evaluation was conducted as in Example 1-1. The results are shown below.

| Sample | Example 1-3 |
| --- | --- |
| Kink occurrence | 0/20 |
| Breakage load | 2.7 kg |
| Flexibility | ◎ |

Comparative Example 1-1

The catheter main segment 2 was produced by repeating the procedure of Example 1-1 except that no reinforcement yarn 51 was provided. The evaluation was conducted as in Example 1-1. The results are shown below.

| Sample | Comparative Example 1-1 |
| --- | --- |
| Kink occurrence | 8/20 |
| Breakage load | 1.2 kg |
| Flexibility | ○ |

Reference Example 1-1

The catheter main segment 2 was produced by repeating the procedure of Example 1-1 except that the number of intersecting points in the braid within one full turn of the reinforcement yarn 51 was increased to 64. The evaluation was conducted as in Example 1-1. The results are shown below.

| Sample | Reference Example 1-1 |
| --- | --- |
| Kink occurrence | 0/20 |
| Breakage load | 2.7 kg |
| Flexibility | ✕ |

Results

When the surface of the reinforcement layer 5 comprising the material of the Examples was heated in the course of the production, the reinforcement yarn 51 partly melted at the temperature of 180°C, while heating of the reinforcement yarn 51 was insufficient at the temperature of 100°C resulting in poor adhesion between the inner and outer layers.

As shown in the Examples, Comparative Example and Reference Example, the catheter main segment 2 used in the intravascular catheter of the present invention showed superiority in all of the frequency of kink occurrence, breakage (damage) resistance, and flexibility. In the additional experiments wherein the catheter main segments 2 were fabricated by repeating the procedures described in the Examples except for the tilt angle of the reinforcement yarn 51 in relation to the longitudinal axis of the catheter main segment 2, number of the reinforcement yarns 51, and the like, the catheter main segments 2 fabricated exhibited excellent properties comparable to those of the Examples.

Example 2

The catheter main segments 2 of the structures shown in FIGS. 2 and 3 were produced by repeating the procedure of Example 1-1 except that the temperature of the preliminary heating and the number of reinforcement yarns 51 were changed.

In the Examples and Comparative Examples, below, surface of the reinforcement layer 5 was heated to 200 to 200°C after the continuous winding of the reinforcement yarns 51 for embedding of the reinforcement yarns 51 in the inner layer 4. The degree of the embedding was such that, in the cross section of the catheter main segment 2 in the plane perpendicular to the longitudinal axis of the catheter main segment 2, the reinforcement layer 5 was embedded in the inner layer 4 that the total cross sectional area of the reinforcement layer 5 embedded in the inner layer 4 was in the range of 30 to 50% of the total cross sectional area of the reinforcement layer 5.

Example 2-1

The catheter main segment 2 was produced in accordance with the specifications and the production conditions as described below.

Total length of the catheter main segment 2: 32 mm
Outer diameter of the outer layer 6: 0.86 mm
Outer diameter of the inner layer 4: 0.75 mm
Inner diameter of the inner layer 4: 0.64 mm

Material of the outer layer 6: thermoplastic polyurethane elastomer (Shore D hardness: 67, Melting point To: 170°C)

Material of the inner layer 4: the one used for the outer layer (Melting point Ti: 170°C)

Material of the reinforcement yarn 51: polyamide (nylon 6, melting point Tt: 210°C), single filament
Diameter of the reinforcement yarn 51: 41 μm
Tilt angle θ of the reinforcement yarn 51 in relation to the longitudinal axis of the catheter main segment: 30°
Number of the reinforcement yarns 51: 16

Temperature Tp to which the surface of the reinforcement layer 5 is heated: 200°C
Degree of embedding of the reinforcement layer 5: 30%

The resulting catheter main segment 2 was evaluated as described below.

Kink resistance and Breakage resistance

The kink resistance and the breakage resistance at room temperature (20°C) were evaluated as described in Example 1.

Surface smoothness

The surface smoothness of the catheter main segment 2 was evaluated by touching the surface with finger, and by measuring surface roughness Rz in accordance with JIS.

The surface roughness Rz (average of 10 points) was measured in accordance with JIS, and more illustratively, the surface roughness Rz was calculated for a standard length of the surface profile, as a difference in micrometer (pm) between average height of the highest to the fifth highest peaks and average height of the bottoms of the deepest to the fifth deepest valleys where the height is measured in transverse direction from a line which is parallel to the mean line and which does not intersect the surface profile. The results were as shown below.

| Sample | Example 2-1 |
|---|---|
| Kink occurrence | 0/20 |
| Breakage load | 2.7 kg |
| Surface smoothness | good |
| Rz (average of 10 points) | 3.72 |

Example 2-2

The catheter main segment 2 was produced by repeating the procedure of Example 2-1 except that the number of reinforcement yarns 51 was changed to 12. The evaluation was conducted as in Example 2-1. The results are shown below.

| Sample | Example 2-2 |
|---|---|
| Kink occurrence | 0/20 |
| Breakage load | 2.3 kg |
| Surface smoothness | good |
| Rz (average of 10 points) | 3.65 |

Example 2-3

The catheter main segment 2 was produced by repeating the procedure of Example 2-1 except that the surface of the reinforcement layer 5 was heated at a temperature (Tp) of 220°C. The evaluation was conducted as in Example 2-1. The results are shown below.

| Sample | Example 2-3 |
|---|---|
| Kink occurrence | 0/20 |
| Breakage load | 2.7 kg |
| Surface smoothness | good |
| Rz (average of 10 points) | 3.68 |

Example 2-4

The catheter main segment 2 was produced by repeating the procedure of Example 2-1 except that 50% of the total cross sectional area of the reinforcement layer 5 was embedded in the inner layer 4. The evaluation was conducted as in Example 2-1. The results are shown below.

| Sample | Example 2-4 |
|---|---|
| Kink occurrence | 0/20 |
| Breakage load | 2.7 kg |
| Surface smoothness | excellent |
| Rz (average of 10 points) | 2.47 |

Comparative Example 2-1

The catheter main segment 2 was produced by repeating the procedure of Example 2-1 except that no reinforcement yarn 51 was provided. The evaluation was conducted as in Example 2-1. The results are shown below.

| Sample | Comparative Example 2-1 |
| --- | --- |
| Kink occurrence | 8/20 |
| Breakage load | 1.2 kg |
| Surface smoothness | excellent |
| Rz (average of 10 points) | 1.13 |

Reference Example 2-1

The catheter main segment 2 was produced by repeating the procedure of Example 2-1 except that the surface heating of the reinforcement layer 5 was omitted and no reinforcement yarn 51 was provided. The evaluation was conducted as in Example 2-1. The results are shown below.

| Sample | Reference Example 2-1 |
| --- | --- |
| Kink occurrence | 3/20 |
| Breakage load | 2.2 kg |
| Surface smoothness | very poor |
| Rz (average of 10 points) | 13.49 |

Reference Example 2-2

The catheter main segment 2 was produced by repeating the procedure of Example 2-1 except that 20% of the total cross sectional area of the reinforcement layer 5 was embedded in the inner layer 4. The evaluation was conducted as in Example 2-1. The results are shown below.

| Sample | Reference Example 2-2 |
| --- | --- |
| Kink occurrence | 1/20 |
| Breakage load | 2.4 kg |
| Surface smoothness | poor |
| Rz (average of 10 points) | 12.13 |

Reference Example 2-3

The catheter main segment 2 was produced by repeating the procedure of Example 2-1 except that the 90% of the total cross sectional area of the reinforcement layer 5 was embedded in the inner layer 4. The evaluation was conducted as in Example 2-1. The results are shown below.

| Sample | Reference Example 2-3 |
| --- | --- |
| Kink occurrence | 0/20 |
| Breakage load | 2.4 kg |
| Surface smoothness | poor (exterior and inner surfaces) |
| Rz (average of 10 points) | 9.44 |

Results

When the surface of the reinforcement layer 5 was heated in the course of the production, the reinforcement yarn 51 melted at the temperature of 240°C, and the merit of providing the reinforcement layer 5 was almost nulled. On the other hand, heating at 170°C proved insufficient and the reinforcement yarns 51 hardly became embedded in the inner layer 4.

As shown in the Examples, Comparative Example and Reference Examples, the catheter main segment 2 used in the intravascular catheter of the present invention showed superiority in all of the frequency of kink occurrence, breakage (damage) resistance, and flexibility. In the additional experiments wherein the catheter main segments 2 were fabricated by repeating the procedures described in the Examples except for the tilt angle of the reinforcement yarn 51 in relation to the longitudinal axis of the catheter main segment 2, number of the reinforcement yarns 51, and the like, the catheter main segments 2 fabricated exhibited excellent properties comparable to those of the Examples.

Example 3

The catheter main segments 2 of the structures shown in FIGS. 2 and 3 were produced.

The reinforcement 5 was formed by using a power-driven braider, and the reinforcement yarns 51 were successively wound in alternate directions by densely supplying the yarns from the dies.

More illustratively, the winding was accomplished by supplying the reinforcement yarn 51 from a yarn supplying member of one power-driven braider, and longitudinally moving the inner layer 4 in relation to said yarn supplying member simultaneously with the rotation of the inner layer 4 around its longitudinal axis to thereby continuously wind the reinforcement yarn 51 on the exterior surface of the inner layer 4 at a constant angle in relation to the longitudinal axis of the catheter main segment 2 and at a constant pitch. Successively, another reinforcement yarn 51 was supplied from a yarn supplying member of another power-driven braider machine, and the inner layer 4 was longitudinally moved in relation to said yarn supplying member simultaneously with the rotation of the inner layer 4 around its longitudinal axis in the reverse direction to thereby continuously wind the reinforcement yarn on the exterior surface of the inner layer 4 at a constant angle in relation to the longitudinal axis of the catheter main segment 2 and at a constant pitch. As a consequence, the reinforcement yarn 51 wound in one direction intersected with the reinforcement yarn 51 wound in another direction, and intersecting points were formed. By setting an adequate number of reinforcement yarns 51 in the yarn supplying member, number of reinforcement yarns 51 in one pitch, namely, density of the reinforcement yarns 51 could be determined.

Before the coating of the material of the outer layer 6 in molten state, the surface of the reinforcement layer 5 was preliminarily heated to 210°C to adequately heat the reinforcement yarn 51. The material of the outer layer 6 was then coated, and the coating was cooled for solidification to thereby form the outer layer 6.

Example 3-1

The catheter main segment 2 was produced in accordance with the specifications and the production conditions as described below.

Total length of the catheter main segment 2: 32 mm
Outer diameter of the outer layer 6: 0.86 mm
Outer diameter of the inner layer 4: 0.75 mm
Inner diameter of the inner layer 4: 0.64 mm

Material of the outer layer 6: thermoplastic polyurethane elastomer (Miractoran (E568 PNAT) manufactured by Nippon Miractoran K.K.) (Shore D hardness: 68; Temperature To at which the melt viscosity reaches 100,000 poise: 208°C; Vicat softening temperature Tb: 141°C)

Material of the inner layer 4: the one used for the outer layer (Temperature Ti at which the melt viscosity reaches 100,000 poise: 208°C; Vicat softening temperature Tb: 141°C)

Material of the reinforcement yarn 51: polyamide (nylon 6, melting point Tt: 221°C), single filament
Diameter of the reinforcement yarn 51: 41 $\mu$m
Radiopaque substance 52: barium sulfate (average particle diameter: 0.65 $\mu$m)
Structure of the reinforcement layer 5: braid of the reinforcement yarns 51 (the structure of FIG. 3)
Tilt angle $\theta$ of the reinforcement yarn 51 in relation to the longitudinal axis of the catheter main segment: 45°
Winding pitch: 1.5 mm
Number of the reinforcement yarns 51: 16
Temperature Tp of preliminary heating: 210°C

The resulting catheter main segment 2 was evaluated as described below.

Kink resistance and Breakage resistance

The breakage resistance at room temperature (20°C) and at a constant temperature of 37°C (near body temperature), and the kink resistance were evaluated as described in Example 1.

Surface smoothness

The surface smoothness was evaluated as described in Example 2.

Radiopacity

The radiopacity was evaluated by visually inspecting the radiograph. The results were as shown below.

| Kink occurrence | 0/20 |
|---|---|
| Breaking load<br>at room temp. (20°C)<br>at constant temp (37°C) | <br>2.7 kg<br>2.0 kg |
| Exterior surface smoothness<br>visual inspection<br>Rz (average of 10 points) | <br>good<br>3.01 |
| Radiopacity | excellent |

Example 3-2

The catheter main segment 2 was produced by repeating the procedure of Example 3-1 except that the number of reinforcement yarns 51 was changed to 12. The evaluation was conducted as in Example 3-1. The results are shown below.

| Kink occurrence | 0/20 |
|---|---|
| Breaking load<br>at room temp. (20°C)<br>at constant temp (37°C) | <br>2.3 kg<br>1.7 kg |
| Exterior surface smoothness<br>visual inspection<br>Rz (average of 10 points) | <br>good<br>2.53 |
| Radiopacity | good |

Example 3-3

The catheter main segment 2 was produced by repeating the procedure of Example 3-1 except that the radiopaque substance 52 was changed to bismuth oxide (average particle diameter, 2.5 μm). The evaluation was conducted as in Example 3-1. The results are shown below.

| Kink occurrence | 0/20 |
|---|---|
| Breaking load<br>at room temp. (20°C)<br>at constant temp (37°C) | <br>2.6 kg<br>2.0 kg |
| Exterior surface smoothness<br>visual inspection<br>Rz (average of 10 points) | <br>good<br>3.18 |
| Radiopacity | excellent |

Example 3-4

The catheter main segment 2 was produced by repeating the procedure of Example 3-1 except that the radiopaque substance 52 was incorporated in the reinforcement yarn 51 as a core constituting 50% of the cross sectional area of the reinforcement yarn 51. The evaluation was conducted as in Example 3-1. The results are shown below.

| Kink occurrence | 0/20 |
|---|---|
| Breaking load | |
|     at room temp. (20°C) | 2.8 kg |
|     at constant temp (37°C) | 2.1 kg |
| Exterior surface smoothness | |
|     visual inspection | good |
|     Rz (average of 10 points) | 3.16 |
| Radiopacity | good |

Comparative Example 3-1

The catheter main segment 2 was produced by repeating the procedure of Example 3-1 except that no reinforcement yarn 51 was provided on the inner layer 4. The evaluation was conducted as in Example 3-1. The results are shown below.

| Kink occurrence | 8/20 |
|---|---|
| Breaking load | |
|     at room temp. (20°C) | 1.2 kg |
|     at constant temp (37°C) | 0.6 kg |
| Exterior surface smoothness | |
|     visual inspection | good |
|     Rz (average of 10 points) | 0.98 |
| Radiopacity | none |

Comparative Example 3-2

The catheter main segment 2 was produced by repeating the procedure of Example 3-1 except that 30% by weight of barium sulfate was homogeneously blended in the material constituting the inner and outer layers 4 and 6. The evaluation was conducted as in Example 3-1. The results are shown below.

| Kink occurrence | 9/20 |
|---|---|
| Breaking load | |
|     at room temp. (20°C) | 1.1 kg |
|     at constant temp (37°C) | 0.6 kg |
| Exterior surface smoothness | |
|     visual inspection | poor (rough) |
|     Rz (average of 10 points) | 1.23 |
| Radiopacity | good |

Reference Example 3-1

The catheter main segment 2 was produced by repeating the procedure of Example 3-1 except that the preliminary heating was conducted at the temperature Tp of 230°C. The evaluation was conducted as in Example 3-1. The results are shown below.

| Kink occurrence | 6/20 |
|---|---|
| Breaking load at room temp. (20°C) at constant temp (37°C) | 1.5 kg 0.8 kg |
| Exterior surface smoothness visual inspection Rz (average of 10 points) | good 1.17 |
| Radiopacity | good |

Reference Example 3-2

The catheter main segment 2 was produced by repeating the procedure of Example 3-1 except that the preliminary heating was conducted at the temperature Tp of 170°C. The evaluation was conducted as in Example 3-1. The results are shown below.

| Kink occurrence Breaking load | 6/20 |
|---|---|
| at room temp. (20°C) at constant temp (37°C) | 2.6 kg 2.0 kg |
| Exterior surface smoothness visual inspection Rz (average of 10 points) | good 3.39 |
| Radiopacity | excellent |

Reference Example 3-3

The catheter main segment 2 was produced by repeating the procedure of Example 3-2 except that the preliminary heating was conducted at the temperature Tp of 230°C. The evaluation was conducted as in Example 3-2. The results are shown below.

| Kink occurrence | 7/20 |
|---|---|
| Breaking load at room temp. (20°C) at constant temp (37°C) | 1.3 kg 0.7 kg |
| Exterior surface smoothness visual inspection Rz (average of 10 points) | good 2.39 |
| Radiopacity | good |

Reference Example 3-4

The catheter main segment 2 was produced by repeating the procedure of Example 3-1 except that the preliminary heating was conducted at the temperature Tp of 180°C, and the inner layer 4 and the outer layer 6 were produced from a thermoplastic polyurethane elastomer having Shore D hardness of 74, the temperature To at which the melt viscosity reaches 100, 000 poise of 220°C, and Vicat softening temperature Tb of 151°C. The evaluation was conducted as in Example 3-1. The results are shown below.

| Kink occurrence | 4/20 |
|---|---|
| Breaking load at room temp. (20°C) | 2.6 kg |

(continued)

| | |
|---|---|
| at constant temp (37°C) | 1.9 kg |
| Exterior surface smoothness<br>visual inspection<br>Rz (average of 10 points) | <br>good<br>3.24 |
| Radiopacity | good |

Reference Example 3-5

The catheter main segment 2 was produced by repeating the procedure of Example 3-1 except that the preliminary heating was conducted at the temperature Tp of 185°C, and the inner layer 4 and the outer layer 6 were produced from a thermoplastic polyurethane elastomer having Shore D hardness of 76, the temperature To at which the melt viscosity reaches 100,000 poise of 225°C, and Vicat softening temperature Tb of 154°C. The evaluation was conducted as in Example 3-1. The results are shown below.

| | |
|---|---|
| Kink occurrence | 5/20 |
| Breaking load<br>at room temp. (20°C)<br>at constant temp (37°C) | <br>2.5 kg<br>1.8 kg |
| Exterior surface smoothness<br>visual inspection<br>Rz (average of 10 points) | <br>good<br>3.41 |
| Radiopacity | good |

Results

When the surface of the reinforcement layer 5 was heated in the course of the production, the reinforcement yarn 51 melted at the temperature of 230°C, and the merit of providing the reinforcement layer 5 was almost nulled. On the other hand, heating temperature of 170°C proved too low to sufficiently heat the inner layer 4 and the reinforcement yarns 51 to enable good adhesion between the inner and outer layers 4 and 6.

As shown in the Examples, Comparative Example and Reference Example, the catheter main segment 2 used in the intravascular catheter of the present invention showed superiority in all of the frequency of kink occurrence, breakage (damage) resistance, lubricity, and radiopacity.

In the additional experiments wherein the catheter main segments 2 were fabricated by repeating the procedures described in the Examples except that the reinforcement yarns 51 contained the radiopaque substance as their core were used, the catheter main segments 2 fabricated exhibited excellent properties comparable to those of the Examples.

It should be noted that the present invention is by no mean limited to the above described Examples which are given by way of examples.

MERITS OF THE INVENTION

As described above, the intravascular catheter and the intravascular catheter assembly of the present invention which are provided with the reinforcement layer have a sufficient strength, and as a consequence, an improved breakage resistance and kink resistance could be realized simultaneously with an appropriate flexibility.

In particular, when reinforcement yarns were used to constitute the reinforcement layer, flexibility of the catheter main segment and the reinforcement effect of the reinforcement yarn could be adequately adjusted. In addition, by limiting the tilt angle of the reinforcement yarn in relation to the longitudinal axis of the catheter main segment, an improved kink resistance as well as trackability could be provided with the catheter main segment. Synergistic effects were also realized by limiting the area ratio of the reinforcement layer in the cross sectional area of the catheter main segment.

When a thermoplastic resin such as a polyamide resin was used for the reinforcement yarn, the reinforcement yarn in the tip portion of the catheter main segment melted or softened to facilitate the thermal working of the tip portion.

Such improvement in the workability of the tip portion contributed for the prevention of the damage of the catheter main segment.

By constituting the reinforcement layer such that the number of the intersecting points S meets the relation: n x m > S, excellent kink resistance as well as breakage resistance could be realized simultaneously with an adequate flexibility, and as a consequence, the catheter was provided with a sufficient trackability to follow the curves and the bends of the blood vessel as well as sufficient kink resistance and flexibility to prevent kink and folding at such curves and bends.

Furthermore, since the reinforcement is partly embedded in the inner layer, surface smoothness as well as adhesion between the inner and outer layers have been improved to result in the improved kink resistance. In addition, such improved adhesion between the inner and outer layers have also prevented the reinforcement from displacement. As a consequence, accidents such as breakage and kink of the catheter main segment, and hence, damage of the blood vessel could be prevented. In other words, in the present invention, presence of the reinforcement partly embedded in the inner layer has enabled improved kink resistance and breakage resistance as well as excellent surface smoothness to be realized at once. Therefore, trackability at the curves and bends of the blood vessel was ensured together with high kink resistance and good slidability to prevent kink and folding at such sites of the blood vessel.

In the embodiments wherein a reinforcement yarn containing a radiopaque substance in its interior is used for the reinforcement, high radiopacity was readily realized with no increase in the wall thickness owing to the radiopacity of the reinforcement yarn. In addition, if the catheter main body should be damaged in the body to leave broken pieces in the body despite the high strength of the catheter main segment against the damage by the acute needle edge, search of the broken pieces by radiography is facilitated by the radiopacity. Despite such radiopacity, the intravascular catheter has sufficient surface smoothness as well as safely since the radiopaque substance is not exposed.

**Claims**

1. An intravascular catheter having a tubular, flexible catheter main segment wherein said catheter main segment comprises an inner layer, a reinforcement layer formed from reinforcement yarns disposed on the inner layer, and an outer layer, wherein

   said catheter main segment has at least a part wherein said inner and outer layers are adhered to each other via said reinforcement layer, and
   said reinforcement layer comprises a braid of reinforcement yarns wherein the yarns are wound in spirals of two directions, and wherein the relation:

$$n \times m > S$$

   is met when n is the number of the reinforcement yarns wound in one direction,
   m is the number of the reinforcement yarns wound in another direction, and
   S is the total number of intersecting points in the braid within one full turn of the reinforcement yarn.

2. An intravascular catheter having a tubular, flexible catheter main segment wherein said catheter main segment comprises an inner layer, a reinforcement layer formed from reinforcement yarns disposed on the inner layer, and an outer layer, wherein

   said catheter main segment has at least a part wherein said inner and outer layers are adhered to each other via said reinforcement layer, and
   said reinforcement yarns are partly embedded in the inner layer of said catheter main segment in the cross section perpendicular to axial direction of said catheter.

3. An intravascular catheter having a tubular, flexible catheter main segment wherein said catheter main segment comprises an inner layer, a reinforcement layer formed from reinforcement yarns disposed on the inner layer, and an outer layer, wherein

   said catheter main segment has at least a part wherein said inner and outer layers are adhered to each other via said reinforcement layer, and
   the relation:

$$Tt > Ti + 5°C$$

is met when
Tt is melting temperature of said reinforcement yarns, and
Ti is the temperature at which the material constituting said inner layer reaches down to a melt viscosity of 100,000 poise.

4. An intravascular catheter according to any one of claims 1 to 3 wherein said flexible material is a thermoplastic elastomer.

5. An intravascular catheter according to claim 4 wherein said thermoplastic elastomer is selected from polyamide elastomers and polyurethane elastomers.

6. An intravascular catheter according to any one of claims 1 to 5 wherein said reinforcement yarn comprises a member selected from polyamide thermoplastic resins, polyester thermoplastic resins, and mixtures of such resins with an inorganic material.

7. An intravascular catheter according to any one of claims 1 to 6 wherein the exterior surface of the outer layer has a surface roughness Rz (measured in accordance with JIS) of up to 7 $\mu$m.

8. An intravascular catheter according to any one of claims 1 to 7 wherein said catheter main segment has an inner diameter of about 0.4 to about 1.8 mm, an outer diameter of about 0.6 to about 2.3 mm, and a total wall thickness of about 0.08 to about 0.5 m.

9. An intravascular catheter according to claim 2 wherein 30 to 80% in total in cross sectional area of the reinforcement is embedded in the inner layer.

10. An intravascular catheter according to claim 3 wherein said reinforcement yarns contain a radiopaque substance.

11. An intravascular catheter according to claim 1 wherein the number of the reinforcement yarns is such that n $\geq$ 3 and m $\geq$ 3.

12. An intravascular catheter according to claim 1 wherein the number of the reinforcement yarns is such that n = m.

13. An intravascular catheter according to claim 1 wherein the number S of intersecting points in one turn of the reinforcement yarn is preferably such that n x m > 1.5 x S.

14. A method for producing the intravascular catheter according to any one of claims 1 to 3 comprising the steps of forming the inner layer of tubular form, providing the reinforcement layer comprising the reinforcement yarns on the exterior surface of said tubular inner layer, and coating the exterior surface of the inner layer and the reinforcement layer with the material of the outer layer in molten state, wherein, before such coating, the tubular inner layer having the reinforcement layer provided thereon is preliminarily heated to a degree such that the reinforcement yarn retains its shape.

15. An intravascular catheter assembly comprising the intravascular catheter according to any one of claims 1 to 13 and a needle inserted in said intravascular catheter.

FIG.1

## FIG.2

## FIG.3

# FIG.4

# FIG.5

# FIG.6

51

52

# FIG.7

51

52

# FIG.8

# FIG.9